Europäisches Patentamt

European Patent Office

Office européen des brevets

(19)

(11) Publication number: **0 238 053 B1**

# EUROPEAN PATENT SPECIFICATION

(45) Date of publication of patent specification: **27.12.91**    (51) Int. Cl.⁵: **A61F 13/15**

(21) Application number: **87103918.6**

(22) Date of filing: **18.03.87**

(54) Anatomic absorbent.

(30) Priority: **19.03.86 BR 8601228**

(43) Date of publication of application:
**23.09.87 Bulletin 87/39**

(45) Publication of the grant of the patent:
**27.12.91 Bulletin 91/52**

(84) Designated Contracting States:
**BE DE ES GB IT LU NL SE**

(56) References cited:
**EP-A- 0 091 412        EP-A- 0 119 825**
**EP-A- 0 155 515        EP-A- 0 167 931**
**DE-A- 2 854 792        FR-A- 2 513 115**

(73) Proprietor: **McNEIL-PPC, Inc.**
**Van Liew Avenue**
**Milltown New Jersey 08850(US)**

(72) Inventor: **Everson Amaral, Rua Bernardo**
**Grabois**
**271 Jardim Das Induatrias**
**Sao Jose Dos Campos Sao Paolo(BR)**

(74) Representative: **Strehl, Schübel-Hopf, Groen-**
**ing**
**Maximilianstrasse 54 Postfach 22 14 55**
**W-8000 München 22(DE)**

EP 0 238 053 B1

## Description

The present invention refers to disposable hygienic absorbents for external use. The prior art has attempted to optimize the efficiency of such disposable products using static concepts in the design of the absorbent substrate, the shape of the pulp body and the improvement of the wrappings. More recently, modifications were proposed with a view to producing a product having an anatomic shape, examples thereof being the absorbents described in French Patent n° 2513115 and in Brazilian Patent Application n° PI-8500769. In the case of the French Patent, the anatomic shape is obtained by a longitudinal curve of the total product and a special shape on the upper absorbent substrate. The product of the Brazilian Patent Application on the other hand comprises an absorbent substrate sandwiched between an impermeable layer designed to be remote from the body of the user and a fluid permeable layer for surface contact in the perineal area of the user. The layers are joined to each other completely around the product along a line spaced inwardly from the peripheral edge thereof. In the central part of the side of the product and outside said sealing line, there is a length of elastic which forces the ends of the absorbent upwardly and towards each other, and the sides inwardly so as to form a cup-shaped profile.

The object of the above-mentioned Brazilian patent application PI-8500769 is to make the absorbent product anatomic whereby it becomes more comfortable to use and less susceptible to leakage. In spite of this, two serious disadvantages have been observed.

Firstly, the shape is not truly anatomic, and could be called pseudo-anatomic, since the elastic strips do not permit flexios which suitably follow the natural movement of the body of the user. Consequently, only relative comfort is provided.

Secondly, the existence of the sealing line between the inner and outer layers which precisely follows the contour of the absorbent substrate and is disposed internally of the elastic strips, creates a non-absorbent strip. Thus when the fluid flow is excessive and there is not time for it to be absorbed by the absorbent substrate through the permeable layer, an accumulation of fluid is formed on the above-mentioned nonabsorbent strip. In such conditions, there is the risk of fluid leaking over the edges of the product or of producing a feeling of discomfort.

With respect to the first disadvantage mentioned above, it will be understood that the development was seeking an anatomic shape in the initial static conditions of use of the product, such shape, however, being lost in dynamic conditions.

The present invention has the purpose of overcoming, at least to a great extent, the above-mentioned disadvantages.

According to the invention, an elongate anatomic absorbent comprises an absorbent substrate sandwiched between a fluid impermeable layer designed to be remote from the body of the user, and a fluid permeable layer for surface contact in the perineal area of the user. These layers are sealed to each other at least at the ends of the absorbent and the sides of the absorbent are provided with elastic means to produce a curved anatomic outline, the absorbent being characterized by the fact that each said elastic means comprises a strip of heat-shrinkable material in the contracted state, so that the sides of the absorbent assume the shape of flaps lifted with relation to horizontal, said layers being adhered to the strips at a plurality of regions inter-spaced along the lengths of strips, the seal between the layers not being continuous in those parts of their flaps which are provided with the strips.

Preferably the absorbent substrate comprises a first layer in contact with the permeable layer, which has characteristics of resilience; a second central layer serving as a fluid reservoir; and a third layer in contact with the impermeable layer, said three layers of the substrate being successively shorter.

The invention is, therefore, based on the used of heat-shrinkable type elastic material at the edges of the products between the permeable and impermeable layers as known from EP-A-0 119 825, so that, after heat-shrinkage, the product acquires a pseudo-anatomic shape. The characteristics of the heat-shrinkable material are considerably different from those of common elastic, as used in the prior art, since elastic forces are uniformly distributed along the length and across the width of the shrinkable strips. As a result, the sides of the absorbent product take the form of upwardly and inwardly turned slightly elastic flaps, which anatomically accompany the movements of the body of the user.

Apart from this, the fact that the bond between the permeable and impermeable layers and the strips of heat-shrinkable material is discontinuous along the strips, results in a slight wrinkling and raising of the flaps along the sides of the absorbent substrate after the heat-shrinkage, which further increases comfort and moreover permits any excess fluid not immediately absorbed and tending to collect in the channel defined around the periphery of the absorbent substrate, to be absorbed in this region because of the absence of continuous sealing. If liquid collects in the wrinkled area, it may for the same reason penetrate the permeable layer and flow downwards to be absorbed by the substrate.

The invention will now be described in greater details by way of example, with reference to the drawings, in which:

Figure 1a is a perspective view of an anatomic absorbent manufactured in accordance with the present invention;

Figure 1b is a schematic representation of the cross-section of the absorbent of figure 1a;

Figure 2 is a cross-section of the absorbent of figure 1 taken along the line 2-2 of figure 3;

Figure 3 is a top view of the product prior to the heat-shrinkage operation;

Figure 4 is a detail of the wrinkling of one side flap after the heat-shrinkage operation;

Figure 5 is a diagramic representation of a longitudinal section of the absorbent substrate; and

Figure 6 is a detail of such substrate.

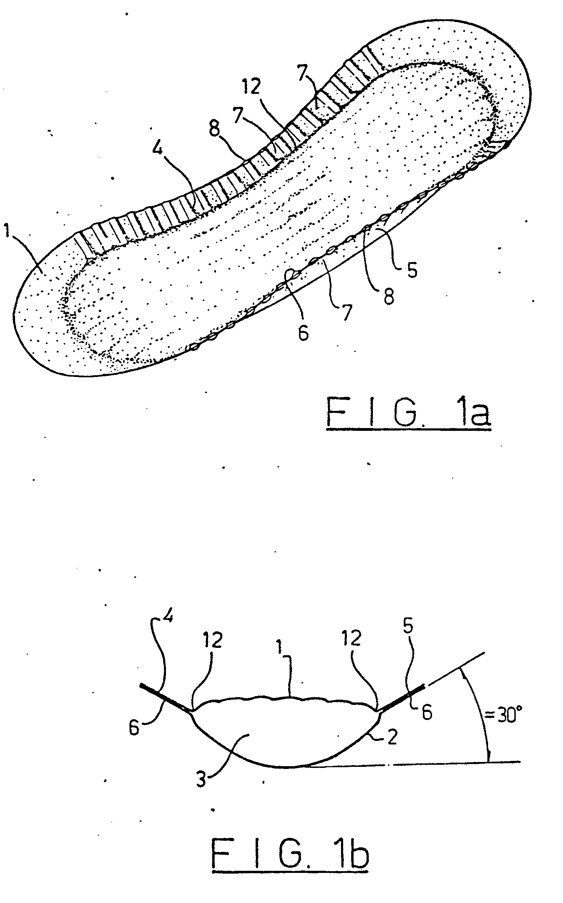

Referring now to Figures 1a and 1b, an anatomic absorbent according to the present invention comprises a fluid permeable layer 1 designed for surface contact with the perineal area of the user; a fluid impermeable layer 2, to be used remote from the body of the user; and sandwiched therebetween, ab absorbent substrate 3.

The absorbent has an elongate shape whose edges laterally of absorbent substrate 3 define flaps 4 and 5 which are raised substantially to continue the bottom profile of the product. Each of the side flaps 4 and 5 comprises not only the two layers 1 and 2, but also sandwiched therebetween along approximately 60% of the length of the product, a heat-shrinkable elastic strip 6 in a contracted state. This elastic strip is bonded to the two layers 1 and 2 along spaced parallel transverse lines, distributed along its length, to produce the wrinkling of the respective flap 4 or 5.

As can be seen from figure 1b, each flap 4 and 5 is raised by the action of the respective strip 6 to define an angle of approximately 30° to the horizontal, which can vary from 10° to about 90°. Said angle as well as the angle of the curve of the bottom surface of the product in the longitudinal direction are not critical, but a curve in the two directions mentioned are necessary to provide the product with its anatomic shape. Moreover, the elasticity provided by flaps 4 and 5 along the sides of the product ensures that. the product is always resiliently and anatomically adaptable to the movements of the user.

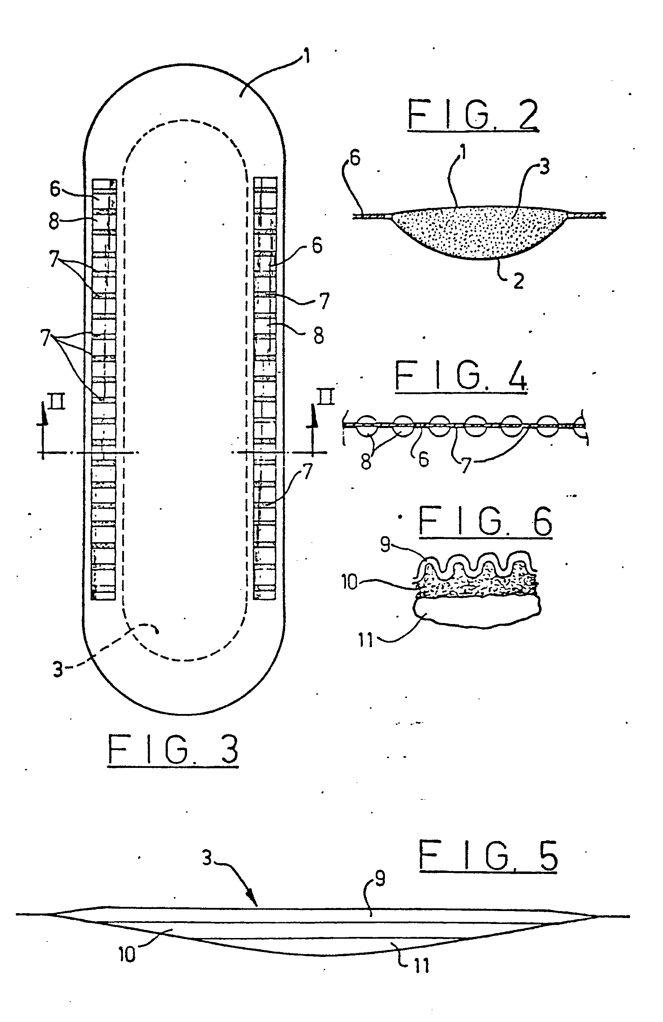

Considering now Figure 3, the product is shown in a top view in a stage of its manufacture immediately prior to the heat-shrinkage operation. Therefore, substrate 1 is visible and therethrough the absorbent substrate 3 and the two heat shrinkable strips 6. Manufacture may then be completed by a heat-crimping machine at a temperature of 80-100° C, preferably 90° C, which applies heat and pressure to the regions indicated by dotted lines 7 in Figure 3. The effect of this operation is to create bonds between each strip 6 and layers 1 and 2 along lines 7 and furthermore provoke heat-shrinkage of strips 6 resulting in the contraction and wrinkling of the side edges of the product to form flaps 4 and 5. This wrinkling may be seen in Figure 4, which also shoes the bonding lines 7 between the two layers 1 and and one of the strips 6, leaving non-bonded free areas 8.

Strips 6 may be made from any suitable heat-shrinkable material, for example PVC (polyvinyl chloride), EPDM (ethylene-propylene-diene monomer) or any other similar material.

It will also be observed that the permeable and impermeable layers 1 and 2 are made from pieces of material cut to have straight parallel sides. In spite of this, it could be advantageous for their sides to be slightly concave.

Figure 5 shows a longitudinal section of absorbent substrate which comprises three layers 9, 10 and 11. The absorbent layer 9 arrange to be in contact with the permeable layer 1, has resilient properties provided by corrugations and embossments.

Figure 6 shows layer 9 corrugated longitudinally of the product. The intermediate absorbent layer 10 is that which actuates as the liquid reservoir and it may or may not contain super absorbent material. The third layer 11, arranged to contact the impermeable layer of the product, actuates as a shape regulator transmitting sensorial characteristics and masking the fluids stored in layer 10, for example by application of liquid repellent, such as PERSISTOL$^R$ commercialized by BASF.

As can be seen from Figure 5, the three layers 9, 10 and 11 are gradually and successively shorter, to collaborate in the obtention of the anatomic shape of the final product.

As seen from Figure 5, the layers 9, 10 and 11 can consist of cellulose fibres of synthetic textile fibres such as rayon, polyester, polypropylene and other fibres. In the case of an intermediate layer 10 made of super absorbent material, said material can be sodium polymethacrylate or any other suitable super absorbent material.

As already mentioned, the absorbent of the present invention has side flaps which, due to the heat-shrinkable strips 6, are raised and have an inherent elasticity which permits them to allow the natural movement of the body of the user, that is to say, permits the product to have an anatomic dynamic action.

Apart from this, any excess of fluid which is not immediately absorbed through the central part of permeable layer 1 and which tends to accumulate in channels 12 (see figure 1a) formed between, on the one hand the inner edges of flaps 4 and 5, and, on the other hand, the main body of the product, is still in contact with a permeable area, in communication with the absorbent substrate 3 due to the absence of a continuous bond line in this region. Experiments have shown that with this configuration the leakage index is lowered by at least 25% when compared with conventional products presently on the market.

It will be understood that the above description is directed to a preferred embodiment of the invention, and that various alterations are possible. It is preferred that the length of the heat-shrinkable strip 6 be approximately 60% of the length of the product. However, said strips may be shorter or longer although it is not advisable that they be less than 40% or greater than 80% of such length. Equally, the manner of effecting the bond between strips 6 and layers 1 and 2 may be varied provided that a continuous bond line is not produced. This and other other modifications are clearly within basic concept of the invention which should be limited only by the scope of the following claims.

## Claims

1. Elongated anatomic absorbent comprising an absorbent substrate sandwiched between a fluid impermeable layer designed to be remote from the body of the user, and a fluid permeable layer for surface contact in the perineal area of the user, said layers being bonded to each other at least at the ends of the absorbent, and the sides of the absorbent having elastic means which provide it with an anatomic curved profile, characterized in that each said elastic means comprises a strip (6) of heat-shrinkable material in the contracted state, so that the sides of the absorbent take the form of flaps (4, 5) raised with relation to the horizontal, said layers (1, 2) being bonded to the strips at a plurality of regions interspaced along the lengths of the strips, the bond between the layers not being continuous in those parts of side flaps (4, 5) which are provided with the strips.

2. Anatomic absorbent according to claim 1, characterized in that the length of each strip covers from 40 to 80% of the respective side of the absorbent.

3. Anatomic absorbent according to claim 2, characterized in that the length of each strip (6) is approximately 60% of the respective side of the absorbent.

4. Anatomic absorbent according to claim 1, 2 or 3, characterized in that each of said layers (1, 2) is made from pieces of material having straight parallel sides.

5. Anatomic absorbent according to claim 1, 2 or 3, characterized in that said absorbent substrate (3) comprises a first layer (9) in contact with said permeable layer, having resilient characteristics; a second central layer (10) serving as a fluid reservoir; and a third layer (11) in contact with the impermeable layer, said three layers of the substrate being successively shorter.

6. Anatomic absorbent according to claim 5, characterized in that said second layer (10) of the absorbent substrate includes super absorbent material.

## Revendications

1. Coussin absorbant de forme anatomique allongé comprenant un substrat absorbant intercalé entre une couche imperméable aux liquides conçue pour être située loin du corps de l'utilisateur, et une couche permable aux liquides destinée à être en contact avec la zone périnéale de l'utilisateur, lesdites couches étant liées l'une à l'autre au moins au niveau des extrémités du coussin absorbant, et les côtés du coussin absorbant ayant des moyens élastiques qui lui donnent un profile de forme anatomique courbée, caractérisé en ce que chacun desdits moyens élastiques comprend une bande (6) de matériau rétrécissant à la chaleur dans son état condensé, afin que les côtés du coussin absorbant prennent la forme de rabats (4, 5) surélevés par rapport à l'horizontale, lesdites couches (1, 2) étant liées aux bandes au niveau d'une pluralité de zones espacées le long des longueurs des bandes, la liaison entre les couches n'étant pas continue au niveau de ces parties de rabats latéraux (4, 5) qui sont fournis avec les bandes.

2. Le coussin absorbant de forme anatomique selon la revendication 1, caractérisé en ce que la longueur de chaque bande couvre de 40 à 80% des côtés respectifs du coussin absorbant.

3. Le coussin absorbant de forme anatomique selon la revendication 2, caractérisé en ce que la longueur de chaque bande (6) représente approximativement 60% des côtés respectifs du coussin absorbant.

4. Le coussin absorbant de forme anatomique selon la revendication 1, 2 ou 3, caractérisé en ce que chacune desdites couches (1, 2) est fabriquée à partir de morceaux de matériau ayant des côtés parallèles droits.

5. Le coussin absorbant de forme anatomique selon la revendication 1, 2 ou 3, caractérisé en ce que ledit substrat absorbant (3) comprend une première couche (9) en contact avec ladite couche perméable, ayant des caractéristiques d'élasticité ; une deuxième couche centrale (10) servant de réservoir de liquides ; et une troisième couche (11) en contact avec la couche imperméable, lesdites trois couches du substrat étant successivement plus courtes.

6. Le coussin absorbant de forme anatomique selon la revendication 5, caractérisé en ce que ladite deuxième couche (10) du substrat absorbant comprend du matériau superabsorbant.

**Patentansprüche**

1. Länglicher, anatomisch geformter Absorptionskörper mit einem absorbierenden Substrat, eingebettet zwischen einer flüssigkeitsundurchlässigen Schicht, die so angeordnet ist, daß sie dem Körper des Benutzers abgekehrt ist, und einer flüssigkeitsdurchlässigen Schicht für den Oberflächenkontakt mit dem Dammbereich des Benutzers, wobei die Schichten zumindest an den Enden des Absorptionskörpers miteinander verbunden sind und die Seiten des Absorptionskörpers elastische Mittel aufweisen, die ihm ein anatomisch gekrümmtes Profil verleihen, **dadurch gekennzeichnet, daß** jedes elastische Mittel einen Streifen (6) aus in der Hitze schrumpfbarem Material im zusammengezogenen Zustand enthält, so daß die Seiten des Absorptionskörpers die Form von Lappen (4, 5) einnehmen, die in bezug auf die Horizontale hochragen, wobei die Schichten (1, 2) an die Streifen in einer Mehrzahl von Bereichen gebunden sind, die in Abständen entlang der Länge der Streifen vorhanden sind, wobei die Verbindung zwischen den Schichten in jenen Teilen der Lappen (4, 5) nicht kontinuierlich ist, die mit den Streifen versehen sind.

2. Anatomisch geformter Absorptionskörper nach Anspruch 1, dadurch gekennzeichnet, daß die Länge jedes Streifens von 40 bis 80 % der jeweiligen Seite des Absorptionskörpers bedeckt.

3. Anatomisch geformter Absorptionskörper nach Anspruch 2, dadurch gekennzeichnet, daß die Länge jedes Streifens (6) annähernd 60 % der jeweiligen Seite des Absorptionskörpers bedeckt.

4. Anatomisch geformter Absorptionskörper nach Anspruch 1, 2 oder 3, dadurch gekennzeichnet, daß jede der Schichten (1, 2) aus Materialstücken mit geraden parallelen Seiten hergestellt ist.

5. Anatomisch geformter Absorptionskörper nach Anspruch 1, 2 oder 3, dadurch gekennzeichnet, daß das absorbierende Substrat (3) enthält: eine erste Schicht (9) mit elastischen Eigenschaften in Kontakt mit der flüssigkeitsdurchlässigen Schicht; eine zweite mittlere Schicht (10), die als Flüssigkeitsreservoir dient; und eine dritte Schicht (11) in Kontakt mit der flüssigkeitsundurchlässigen Schicht, wobei die drei Schichten des Substrats aufeinanderfolgend kürzer sind.

6. Anatomisch geformter Absorptionskörper nach Anspruch 5, dadurch gekennzeichnet, daß die zweite Schicht (10) des absorbierenden Substrats superabsorbierendes Material enthält.

F I G. 1a

F I G. 1b

EP 0 238 053 B1

FIG. 2

FIG. 4

FIG. 6

FIG. 3

FIG. 5

7